# EUROPEAN PATENT APPLICATION

(11) **EP 3 828 260 A1**
(43) Date of publication of application: **02.06.2021**
(21) Application number: 20166497.6
(22) Date of filing: 27.03.2020
(51) Int. Cl.: C12N 1/14, A01G 18/00, E04B 1/14, E04B 1/74, E04C 2/16

(54) **COMPOSITE BIOMATERIAL, OBTAINING METHODS AND USES THEREOF**

(30) Priority: 29.11.2019 PT 2019115945
(71) Applicant: Universidade Católica Portuguesa - UCP, 4169-005 Porto (PT)
(72) Inventor: MARCOS RAMOS, Miguel António, 4440-638 VALONGO (PT); REIS DE SOUSA, Nadine Marisa, 4435-374 RIO TINTO (PT); LIMA CASTRO, Paula Maria, 4169-005 PORTO (PT)
(74) Representative: Patentree

(57) **Abstract**

The present disclosure refers to a new composite material based on woody materials and mycelium, obtaining methods and uses thereof, namely in the field of furniture and coatings.

## Description

### Technical field

The present description refers to a new composite material based on woody materials and mycelium, obtaining methods and uses thereof, namely in the field of furniture and coatings.

### Background

Composite materials based on organic materials and mycelium of saprophytic fungi are highly valuable biomaterials due to their properties with several applications being known. Biomaterial should be understood as a material or a mixture of materials of natural origin which may be biodegradable. Some examples of biomaterials are composites based on coconut fibre, cork, bamboo or bioplastics.

Mycelium, the vegetative growth of fungi, has been used as an aggregating element of organic and mineral materials, creating a connecting network between all the elements, providing resistance while maintaining the lightness of materials. Fungi are composed mainly of a cellular wall, which is constantly growing as it expands in substrates or matrices. Structural oligosaccharides of the fungi cellular wall contain mainly chitin, a strong and stiff substance also found in exoskeletons of arthropods. Chitin is used in several industries as a purified substance for water purification, as food supplements for stabilization, binding agents in fabrics and adhesives, surgical suture wires and medicinal applications.

Currently Portugal has an annual production of about 7 million hL of wine, which corresponds to a vinification of 10000000 ton/year of grapes, taking on the tenth place in the world ranking and the fifth place at a European level (Prozil, 2008; Vitivinicultura, 2007). The wine industry produces high amounts of by-products which, normally, are not valued. The utilization of vinification by-products has, in these last years, deserved the attention of the people responsible for the viticultural politics. The main by-products of the winemaking activity are marc and grape stalk. Grape stalk represents 3-9% of the weight of a bunch of grapes. At a chemical level, it is mainly composed of cellulose (33.4%), lignin (15.5%) and hemicelluloses (14.0%). It is of note the significant amount of tanins (21.4%) and extractables soluble in water (17.4%) (Costa, 2010).

In the last years, fungi mycelium went from being an eligible material for a variety of applications of consumption and construction, being used more and more in alternative to plastics, to other compounds dependent on fossil fuels or to the use of virgin wood. In addition to its resistance and durability, mycelium has many beneficial qualities: it is not toxic, it is resistant to fire, resistant to water and an excellent thermal insulator, among other features. Mycelium may be processed with less energy and materials than the conventional industrial production of composites and may be cultured by environmentally sustainable methods.

Next, we present the documents which we consider that define the state of the art:

Document "Ecovative - 9,914,906" describes a method for solid-state cultivation of mycelium being based on lignocellulosic materials, composed of particles of gymnosperm plant species or species containing resin in their constitution. However, the present invention does not use nor fits to the use of natural materials which contain natural resins as a culture substrate.

Document "Ecovative - 9,795,088" describes the production of an incubated and moulded composite material. In the moulding process, the composite material undergoes an extrusion process in order to create an end product. Another aspect of note is the fact that most of the ingredients used in this process are cereals or small sized organic compounds which allow extrusion. The invention does not use cereals as major ingredient, it gives preference to bigger particles for the interweaving of the final material and does not include extrusion.

Document "Mycoworks, Inc - 20180014468" describes a production method of biomaterials based on the use of mycelium to agglomerate the different particles of the developed formulation, creating objects and structures with different layers. The present invention does not include the production of several layers of material, nor the differentiation of the end product in layers. All the composites that arise from the present invention originate from homogeneous mixtures.

Document "Mycoworks, Inc - 20180148682" describes a moulding system for inoculated lignocellulosic materials in a mould, using specific environmental conditions, applying a compressive strength of at least 10 PSI. The present invention includes a compression process in order to apply the mixtures formulated on the mould. However, the used pressure values are different, using a minimum compressive strength of 800 tons. Another aspect to be considered is the fact that the present invention uses high temperatures (200 °C for the moulding and compression process).

These facts are described in order to illustrate the technical problem addressed by the present invention.

### General description

The present disclosure refers to a composite biomaterial comprising: a woody material as a support; a substrate selected from the following list: seed, cereal, woody material or combinations thereof; a mycelium as an aggregating agent; wherein the woody material is simultaneously support, substrate and inoculum; wherein the mycelium growth aggregates the several components; wherein the mycelium belongs to a species selected from the following list: *Pleurotus ostreatus, Trametes versicolor, Ganoderma lucidum, Fistulina hepatica, Lentinus edodes;* preferably *Pleurotus ostreatus.* The composite material described in the present disclosure is light, uses wastes from another industry and has flexural and/or compressive strength, thus allowing its use in the construction industry and furniture.

In an embodiment for better results, the woody biomaterial is grape stalk.

In an embodiment for better results, the biomaterial comprises 35-60% (v/v) of woody material, preferably 50% (v/v).

In an embodiment for better results, the biomaterial comprises 25-35% (v/v) of seed or cereal, preferably 30% (v/v).

In an embodiment for better results, the biomaterial comprises 10-30% (v/v) of mycelium for inoculation, preferably 20% (v/v).

In an embodiment for better results, the cereal is wheat, rye, oats, barley, ryegrass, or mixtures thereof.

In an embodiment for better results, the seed is a canary seed or rye seed, or mixtures thereof.

In an embodiment for better results, the substrate comprises a mixture of cereal or seed, woody material, calcium carbonate and deionized water.

In an embodiment for better results, the composite biomaterial has fire-retardant properties.

In an embodiment for better results, the composite biomaterial is impermeable.

In an embodiment for better results, the density of the composite material varies between 0.15 - 0.25 g/cm³, preferably from 0.18 - 0.22 g/cm³, more preferably 0.21 g/cm³.

The present disclosure further describes a plate comprising the composite biomaterial described in the present disclosure.

The present disclosure further describes an item comprising the composite biomaterial described in the present disclosure, wherein the item is: logistics object, floor, coating, thermal insulation, acoustic insulation, shelves, furniture, among others.

The present disclosure further describes a composite biomaterial and moulding and thermomoulding methods thereof for application in logistics objects, natural floors or thermal insulation materials.

The material described in the present invention is light, uses wastes from another industry, has flexural and/or compressive strength, thus allowing its use in the construction industry and furniture.

The present disclosure further describes a method of obtaining the material according to any of the preceding claims comprising the following steps: mixing the substrate which comprises cereal or seed with the woody material, wherein the woody material comprises particles 60% smaller than 2 mm, 40% between 2 to 5.6 mm, previously sterilized; inoculating the mycelium (starter culture) in the substrate at 25 °C, for 7 days; subjecting the mixture to a drying process, preferably at 100 °C for 48 h. The method according to claim 15 further comprising a moulding or thermomoulding step.

In an embodiment for better results, the method may comprise the step of placing the mixture in moulds and drying at 25°C for 7 days.

In an embodiment for better results, the method may comprise the step of applying fixation resins according to the following list: epoxy resins, rosin resin, polyurethane or mixtures thereof.

In an embodiment for better results, the mixture is applied over a thermomould.

In an embodiment for better results, the method further comprises an additional step of applying pressure and heat to polymerize the material.

In an embodiment for better results, the moisture of the woody material varies between 40%-60%.

The material described is a composite for application in logistics objects, natural floors or thermal insulation materials.

### Brief description of the drawings

For an easier understanding, drawings are herein attached, which represent preferred embodiments which do not intend to limit the object of the present description.

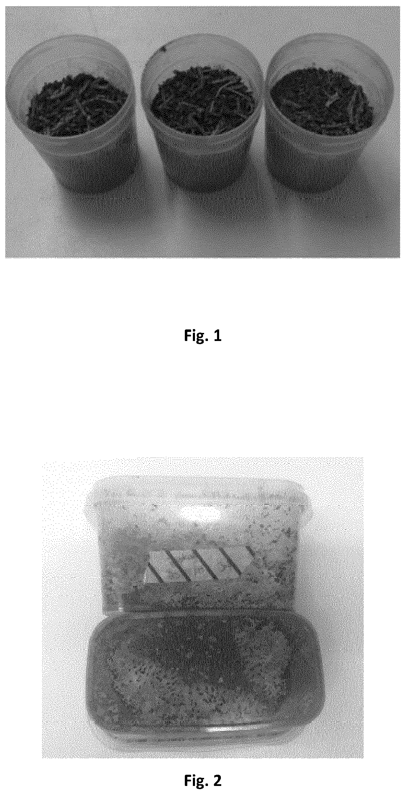

**Figure 1** **-** Mixtures of ground grape stalk.
**Figure 2** **-** Mycelium growth in boxes with micro-aeration.
**Figure 3** - Mould after mycelium growth on the mixtures of grape stalk and canary seed.

### Detailed description

The present description is particularly described using preferred embodiments. Therefore, the disclosure is not limited only to the provided descriptions and illustrations. These are used so that the disclosure is sufficiently detailed and comprehensive. Furthermore, the intent of the drawings is for illustrative purposes and not for limiting purposes.

The present description refers to a new composite biomaterial based on woody materials and mycelium, obtaining methods and uses thereof, namely in the field of furniture and coatings.

The present description refers to the production of composites or biomaterial and moulding methods being based on the use of grape stalk and mycelium as an aggregating agent.

An embodiment of the methodology of the present description comprises the preparation of the woody material. The woody material is dried at room temperature for 5 days until it reaches a moisture ranging between 40%-60%. After this process, the woody material may be stored for later processing.

In an embodiment, the process comprises grinding and sieving steps. Before the grape stalk is ground, it underwent a second drying process. It was placed in a drying chamber at 100 °C for 24 h reaching a moisture value below 10%. For the grinding process, a grinding mill suitable for the grinding of semi-hard products was used. After this process, the ground grape stalk was separated by granulometry using a sieve.

An embodiment of the methodology of the present description comprises culturing mycelium. A substrate was prepared for the mycelium growth composed of an equitable mixture of woody material and cereals. Using propylene boxes with lids containing HEPA filters, 120 mL of a mixture of canary seed and grape stalk, 60 mL deionized water and 2 g calcium carbonate were added. Then, the boxes were subjected to an autoclaving process at 121 °C for 45 minutes. In the end of this step, the substrate was allowed to cool at room temperature. Next, a parcel of mycelium starter culture was taken and the boxes of substrate were inoculated. The boxes were placed in an incubator, at 25 °C, for 7 days so that the substrate was colonized by the mycelium.

In an embodiment, the woody substrate was prepared for the generation of the biomaterial. A new formulation of substrate of mycelium growth was prepared for the generation of the biomaterial. A mixture of particles of grape stalk was selected, having the following granulometry: 60% smaller than 2 mm, 40% between 2 to 5.6 mm. The mixture of grape stalk was posteriorly immersed in 30% hydrogen peroxide for 1 hour. Next, the grape stalk mixture was collected and allowed to drain the excess hydrogen peroxide. A mixture of 30% deionized water and 70% canary seed was autoclaved in a polypropylene bag at 121 °C for 20 minutes.

An embodiment of the methodology of the present description comprises the growth of the mycelium in grape stalk and canary seed on a mould. Using the ingredients prepared in the previous steps, a new mixture was performed having the following volumetric composition: 50% grape stalk mixture (peroxidised), 30% canary seed (autoclaved) and 20% mycelium. This formulation was placed in non-watertight moulds and posteriorly placed in a chamber for 7 days, at 25°C.

In an embodiment, after the mycelium growth on the mould, a new drying or additional dryings are performed. The moulds are placed in the drying chamber, at a temperature of 100 °C, for 48 h.

An embodiment of the methodology of the present description comprises applying fixation resins. After the second drying process, the material is withdrawn from the mould and allowed to cool at room temperature. Next, it was sprayed with epoxy resin (suitable for inside use), polyurethane resin (suitable for outside use) or rosin resins (allows the biodegradability of the material), creating a composite. After the fixation of the resins on the composites, these are allowed to dry at room temperature.

In a preferred embodiment in order to obtain better results, a thermomoulding step applying fixation resins is performed. An alternative to the above described process is to use the previously aggregated mixture of grape stalk, canary seed and mycelium as an ingredient of a new mixture which will be supplemented with fixation resins (epoxy, polyurethane and rosin resin). In this way, it will be possible to apply the latter on a thermomould which by the action of pressure and heat will polymerize the materials creating a moulded composite.

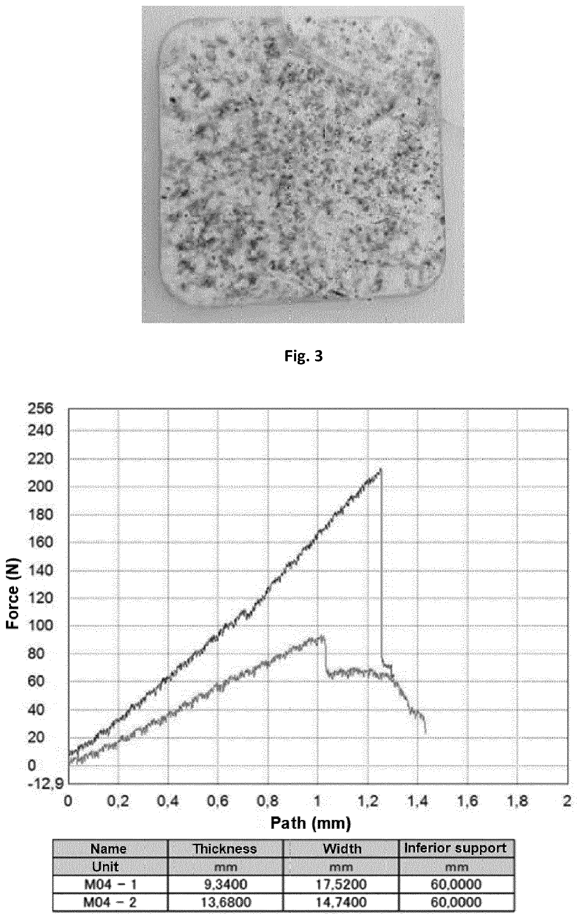

An embodiment of the methodology of the present description comprises mechanical testing. The composites described in the present disclosure presented similar values regardless of the technique of applying fixation resins. The compressive strength test was conducted at a speed of 0.5 mm/minute, presenting varying values between 47-53 N/mm². The presented values are similar when compared to resinous wood (16-58 N/mm²) typically used by the industry in the field of logistics. The reported value of compressive strength for Pinus pinaster wood is 53 N/mm². Regards flexural strength, the test was conducted at the same speed as the one above-mentioned and the values obtained were between 5.5 N/mm² and 6.9 N/mm², showing inferior levels when compared to woods of resinous species. The deforming curve in the flexural test is shown in figure 4.

In an embodiment of the present description, compressive strength tests for the composite material of the present disclosure were conducted.

**Table 1 - Synthesis of the results of the compressive strength tests for the composite material of the present disclosure**

| Name | Max_Force | Max_Tension | Rupture_Force | Rupture_Tension |
|---|---|---|---|---|
| Parameters | Calculus of complete areas | Calculus of complete areas | Sensibility: 10 | Sensibility: 10 |
| Unit | N | N/mm2 | N | N/mm2 |
| 1 | 19608.6 | 52.5837 | -.- | -.- |
| 1 | 19657.7 | 47.7065 | -.- | -.- |
| Average | 19633.2 | 50.1451 | -.- | -.- |
| Standard deviation | 34.7187 | 3.44870 | -.- | -.- |

In an embodiment in order to obtain better results, flexural strength tests were conducted.

**Table 2 - Synthesis of the results of the flexural strength tests for the composite material**

| Name | Max_Force | Rupture_Force | Rupture_Displacing | Max_Tension |
|---|---|---|---|---|
| Parameters | Calculus of complete areas | Sensibility: 10 | Sensibility: 10 | Calculus of complete areas |
| Unit | N | N | mm | N/mm2 |
| M04 - 1 | 93.5523 | -.- | -.- | 5.50895 |
| M04-2 | 213.137 | 211.500 | 1.25473 | 6.95394 |
| Average | 153.345 | 211.500 | 1.25473 | 6.23145 |
| Standard deviation | 84.5592 | -.- | -.- | 1,02176 |

An embodiment of the methodology of the present description comprises physical testing: Flammability test and Impermeability test.

### Flammability testing

In an embodiment, a composite part was obtained having the following dimensions: 20x10x40mm. Next, 10mm of this component were exposed to a 20mm blue flame for 10 seconds. After this period, the flame was withdrawn for verification of the material ignition. The procedure was repeated once again. After these procedures, it was observed that the material did not show any ignition. All the procedure was based on the UL94 Standard for tests for flammability.

### Impermeability testing

A composite plate was used having the following dimensions: 120 x 120 x 15 mm. It was weighted and placed in a 3 L container with deionized water in order to immerse the material for 1 hour. After this procedure, the composite plate was cleaned on the surface using an absorbent material. The weighting step was repeated for verification of changes to the composite plate weight. No weight difference above 0.1% was observed.

From the results above, it is possible to conclude that the composite material of the present invention is a light, flexible material and has compressive and flexural strength.

Naturally, the present invention is not, in any way, limited to the embodiments described in this document and a person skilled in the art may foresee many possibilities of modifications thereof and substitutions of technical features for others which are equivalent, depending on the requirements of each situation, as defined in the appended claims.

The following claims further define preferred embodiments.

### References

1. Bayer, E., McIntyre, G., Swersey; B. Fabricated Panel. U.S 9,795,088, 2017.
2. Costa, V. L. F, Propriedades Papeleiras das fibras do engaço da uva, Departamento de Química, Universidade de Aveiro, Aveiro, 2010, (Tese de Mestrado).
3. Kretschmann, David E. 2010. Mechanical properties of wood. Wood handbook: wood as an engineering material: chapter 5. Centennial ed. General technical report FPL; GTR-190. Madison, WI: U.S. Dept. of Agriculture, Forest Service, Forest Products Laboratory, 2010: p. 5.1-5.46.
4. Pinto, I. (2004). Raw material characteristics of maritime pine (Pinus pinaster Ait.) and their influence on simulated sawing yield: Dissertation. Espoo: VTT.
5. Prozil, S.O., Caracterização Química do Engaço da Uva e Possíveis Aplicações, Departamento de Química, Universidade de Aveiro, Aveiro, 2008, (Tese de Mestrado).
6. Silva, L.M.L.R.d., Caracterização dos Subprodutos da Vinificação. Spectrum, 2003: p.123 - 133.
7. Vitivinicultura - Diagnóstico Sectorial, 2007, Ministério da Agricultura, do Desenvolvimento Rural e das Pescas, (http://www.gpp.pt/images/GPP/O_que disponibilizamos/Publicacoes/Vinh o_Diagnostico_Sectorial.pdf, opened in 24 october 2018)

## Claims

1. A composite biomaterial comprising:
a woody material as a support;
a substrate selected from the following list: seed, cereal, woody material or combinations thereof;
a mycelium as an aggregating agent;
wherein the woody material is simultaneously support, substrate and inoculum;
wherein the mycelium growth aggregates the several components;
wherein the mycelium belongs to a species selected from the following list: *Pleurotus ostreatus, Trametes versicolor, Ganoderma lucidum, Fistulina hepatica, Lentinus edodes;* preferably *Pleurotus ostreatus.*

2. The composite biomaterial according to any of the preceding claims wherein the woody material is grape stalk.

3. The composite biomaterial according to any of the preceding claims comprising 35-60% (v/v) of woody material, preferably 50% (v/v).

4. The composite biomaterial according to any of the preceding claims comprising 25-35% (v/v) of seed or cereal, preferably 30% (v/v).

5. The composite biomaterial according to any of the preceding claims comprising 10-30% (v/v) of mycelium for inoculation, preferably 20% (v/v).

6. The biomaterial according to claim 1 wherein the cereal is wheat, rye, oats, barley, ryegrass, or mixtures thereof; wherein the seed is a canary seed or rye seed, or mixtures thereof.

7. The composite biomaterial according to any of the preceding claims, wherein the substrate comprises a mixture of cereal or seed, woody material, calcium carbonate and deionized water.

8. The composite biomaterial according to any of the preceding claims, wherein the composite material has fire-retardant properties and/or wherein the composite material is impermeable.

9. The composite biomaterial according to any of the preceding claims wherein the density of the composite material varies between 0.15 - 0.25 g/cm³, preferably 0.20 - 0.22 g/cm³.

10. A plate or an item comprising the composite biomaterial described in any of the preceding claims.

11. The plate or item according to the preceding claim wherein the plate or item is: logistics object, floor, coating, thermal insulation, acoustic insulation, shelf, furniture, or combinations thereof.

12. A method of obtaining the biomaterial according to any of the preceding claims comprising the following steps:
mixing the substrate comprising cereal or seed with the woody material, wherein the woody material comprises particles 60% smaller than 2 mm, 40% between 2 to 5.6 mm, previously sterilized;
inoculating the mycelium (starter culture) in the substrate at 25 °C, for 7 days;
subjecting the mixture to a drying process, preferably at 100 °C for 48h;
optionally comprising a moulding or thermomoulding step;
optionally comprising the step of placing the mixture in moulds and drying at 25°C for 7 days;
optionally comprising the step of applying fixation resins according to the following list: epoxy resins, rosin resin, polyurethane or mixtures thereof.

13. The method according to claims 15-17 comprising the step of applying fixation resins according to the following list: epoxy resins, rosin resin, polyurethane or mixtures thereof.

14. The method according to claims 15-19 comprising the step of applying pressure and heat to polymerize the materials.

15. The method according to claims 15-20 wherein the moisture of the woody material varies between 40%-60%.
